Europäisches Patentamt

European Patent Office

Office européen des brevets

(19)

(11) Publication number: **0 278 781 B1**

(12) # EUROPEAN PATENT SPECIFICATION

(45) Date of publication of patent specification: **03.11.93**

(51) Int. Cl.5: **C07K 7/06**, A61K 37/02, A61L 27/00

(21) Application number: **88301198.3**

(22) Date of filing: **12.02.88**

(54) Peptides with laminin activity.

(30) Priority: **12.02.87 US 13919**
**01.10.87 US 102991**

(43) Date of publication of application:
**17.08.88 Bulletin 88/33**

(45) Publication of the grant of the patent:
**03.11.93 Bulletin 93/44**

(84) Designated Contracting States:
**AT BE CH DE FR GB IT LI LU NL SE**

(56) References cited:
**WO-A-84/00540**

**ARCHIVES OF BIOCHEMISTRY AND BIO-PHYSICS, vol. 272, no. 1, pages 39-45, 1989, Academic Press, Inc.; H.K. KLEINMAN et al.: "Identification of a second active site in laminin for promotion of cell adhesion and migration and inhibition of in Vivo melanoma lung colonization"**

(73) Proprietor: **THE UNITED STATES OF AMERICA as represented by the Secretary, United States Department of Commerce National Technical Information Service, Office of Government Inventions and Patents,**
**5285 Port Royal Road**
**Springfield, Virginia 22161(US)**

(72) Inventor: **Martin, George R.**
**5507, Charles Street**
**Bethesda, Maryland 20814(US)**
Inventor: **Sasaki, Makoto**
**2209, Georgian Way No. 43**
**Wheaton, Maryland 20902(US)**
Inventor: **Yamada, Yoshihiko**
**2837, Aquarius Avenue**
**Silver Spring, Maeyland 20906(US)**
Inventor: **Kleinman, Hynda K.,**
**6405, Winston Drive,**
**Bethesda, Maryland 20892(US)**
Inventor: **Robey, Frank**
**8729, Ridge Road**
**Bethesda, Maryland 20817(US)**

EP 0 278 781 B1

BIOCHEMISTRY, vol. 26, 1987, pages 6896-6900, American Chemical Society; J. GRAF et al.: "A pentapeptide from the laminin B1 chain mediates cell adhesion and binds the 67 000 laminin receptor"

Inventor: **Iwamoto, Yukihide**
**2-5-8, Kashii Ekimae**
**Higashi-ku, Fukuoka 813(JP)**
Inventor: **Graf, Jeannette O.,**
**254-35, 75th Avenue,**
**Glen Oaks, New York 11004(US)**

(74) Representative: **Jump, Timothy John Simon et al**
**F.J. Cleveland and Company**
**40-43 Chancery Lane**
**London WC2A 1JO (GB)**

**Description**

FIELD OF THE INVENTION

The present invention is directed to peptides having laminin-like activity.

BACKGROUND OF THE INVENTION

Laminin (Mr = 900,000) is a large glycoprotein specific to basement membranes. Laminin has been shown to promote cell adhesion, cell growth, cell migration, neurite outgrowth, cell differentiation, and to influence the metastatic behavior of tumor cells. Laminin binds to type IV collagen, heparin, gangliosides, and cell surface receptors and promotes the adhesion and growth of various epithelial and tumor cells as well as neurite outgrowth. Laminin is thought to mediate cell-matrix interactions and to be a structural component of all basement membranes binding to collagen IV, heparan sulfate proteoglycan, and nidogen-entactin.

The laminin molecule itself has a cross-like shape when examined by microscopy, with three short arms and one long arm. Two small globules can be observed at the end of each short arm, and a larger globule can be observed at the end of the long arm. Current models suggest that laminin contains one A chain (Mr = 440,000), one B1 chain (Mr = 225,000), and one B2 chain (Mr = 205,000), with part of each chain forming a short arm and the rest of the chain projecting down the long arm.

Laminin exhibits a number of biological activities, including promoting the attachment, migration, and differentiation of certain cells. Some progress has been made in assigning domains in laminin to its activities. Collagen IV binding is attributed to the globules at the end of the short arms. Cell binding is attributed to the portion of laminin minus the long arm and globules. A site in the long arm of laminin is thought to promote axonal outgrowth. Most of the alpha-helical elements in the laminin molecule have been localized to the portion of the long arm adjacent to the terminal globule. The size of the molecule plus difficulty in separating its chains have impeded further characterisation of laminin's structure by conventional chemical approaches.

From Sasaki et al "Sequence of the DNA Encoding the Laminin B1 Chain Reveals a Multi Domain Protein Containing Cysteine Rich Repeats". Proc. Nat. Acad. of Sciences. Vol 84. 1987. Page 935, it is known that active domains have been localized in laminin, based on recent progress is cloning the laminin chains. The B1 chain comprises some 1786 amino acids which appear to form at least six contiguous structural domains. Domains I and II are predominantly alpha-helical and probably extend down the long arm. Domains III and V contain homologous repeats rich in cysteine, and could form rather rigid structures adjacent to the globules formed by domains IV and VI. Studies by the present inventors indicate that a sequence of some five to nine amino acids in domain III is at least partly responsible for the cell attachment, chemotactic, and receptor binding activities of laminin. This sequence also has antimetastatic acitivity with tumor cells.

OBJECTS AND SUMMARY OF THE INVENTION

It is an object of the present invention to provide peptides which have useful biological activity.

It is a further object of the present invention to provide peptides which have the biological activity of laminin.

It is yet a further object of the present invention to provide peptides which have biological activity in the field of cell adhesion and migration and blocking of tumor metastases.

It is yet another object of the present invention to provide peptides which block antiogenesis.

It is yet another object of the present invention to provide peptides which alter the formation of capillary structures by endothelial cells.

It is yet a further object of the present invention to provide peptides which prevent an excess of blood vessels in tissues due to inflammation or other pathological conditions due to Kaposi sarcoma.

Two peptides have been found to have particularly useful properties: a pentapeptide and a nonapeptide, although several other related peptides were nearly as active as the pentapeptide and the nonapeptide.

The pentapeptide of the present invention has the following amino acid sequence:

tyrosine-isoleucine-glycine-serine-arginine.

The nonapeptide of the present invention has the following amino acid sequence:

cysteine-aspartate-proline-glycine-tyrosine-isoleucine-glycine-serine-arginine, i.e., CDPGYIGSR.

3

The entire primary peptide sequence of one of the chains of laminin was determined from cDNA cloning. Using synthetic peptides prepared on a peptide synthesizer, the active domain on the B1 chain responsible for cell attachment and cell migration was identified. Peptides of 20 amino acids and their corresponding antibodies were prepared to each of the seven structural domains. None of these peptides was active, although one of the antibodies blocked cell attachment. Smaller synthetic peptides were prepared to the region around the amino acid sequence specific to this active antibody. A nine amino acid peptide was found to be directly active in cell attachment and cell migration. Various combinations of smaller peptides which authentically matched the protein sequence and/or contained substitutions were tested until the pentapeptide (peptide 5) of the present invention was found to be the most active sequence with the minimal number of amino acids.

DESCRIPTION OF THE DRAWINGS

Figure 1A shows the ability of peptide 5 specific antibodies to react with their corresponding peptides and with laminin.
Figure 1B shows the specificity of the peptide antisera for the B chain of laminin.
Figure 2 shows that the antibody of peptide 5 inhibits laminin-mediated HT1080 cell attachment.
Figure 3 shows that peptide 11 can promote HT 1080 cell attachment when coated onto a plastic dish.
Figure 4 shows that a peptide 11-albumin conjugate can promote HT1080 and CHO cell attachment.
Figure 5 shows the ability of a peptide 11-albumin conjugate to inhibit attachment to laminin.
Figure 6A shows that peptide 11 elutes a 67 Kd protein, comparable in migration position to the laminin receptor from a laminin affinity column to which NG108-15 cell membrane proteins have been applied.
Figure 6B shows a Western blot with anti-laminin receptor antibody reacting with the material eluted from the laminin affinity column by peptide 11.
Figure 7 shows that peptide 11 can inhibit tumor cell invasion in vitro.
Figure 8 shows that YIGSR amide is the smallest active peptide for cell attachment.
Figure 9 shows that YIGSR amide is the smallest active peptide for cell migration.
Figure 10 shows that YIGSR amide is the smallest active peptide for tumor cell invasion.
Figure 11 shows that YIGSR amide is the smallest active peptide for receptor elution.
Figure 12 shows the effect of YIGSR-amide on capillary endothelial cell tube formation.
Figure 13 shows inhibition of invasion by laminin peptides.

DETAILED DESCRIPTION OF THE INVENTION

The peptides studied were synthesized using a commercially available automated peptide synthesizer (Model 430A, Applied Biosystems, Inc., Foster City, California). Deprotection and release of the peptide from the solid phase support matrix were accomplished by treating the protected peptide on the resin with anhydrous HF containing 10% thioanisole or 10% anisole for one to two hours at 0°C. Following deprotection and release from the resin, the peptides were extracted with either ethyl acetate or diethyl ether. The peptides were then dissolved in 20-50 ml of 10% aqueous acetic acid and filtered to remove the resin. The filtrate was lyophilized to yield white to off-white powders. The composition of all of the peptides was verified by amino acid analysis. Peptides suspected of possessing biological activity were further purified using preparative high pressure liquid chromatography (HPLC) when analytical HPLC indicated that this was necessary. Purity of the pure peptides was verified by HPLC and amino acid analysis. Average yields for all syntheses ranged from 80-90%. The peptides were conjugated to bovine serum albumin for immunization.

Prior to conjugating a peptide to bovine serum albumin (BSA), the BSA was first derivatized with a nucleophilic spacer, consisting of adipic dihydrazide (ADH). The resulting derivatized BSA (BSA-ADH) was stored as a dry powder at 4°C until used. For the conjugation reaction, the peptide was reacted with an equi-molar amount of bromoacetylbromide in 5 ml of 0.1M $NaHCO_3$, and the conjugation reaction was allowed to proceed with magnetic stirring for 18 hours at 25°C. Following the conjugation process, the conjugates were passed through a 2.5 x 30 cm column of Sephadex G-50 in 0.1M $NH_4HCO_3$. Those fractions containing protein, as judged by their absorbance at 280 nm, were pooled and lyophilized.

The conjugate was diluted to 1 mg/ml with phosphate buffered saline (PBS) at pH 7.4, and it was mixed with an equal volume of complete Freund's adjuvant for the initial immunization. Rabbits were injected subcutaneously and 14 days later received their second injection. Thereafter, the animals were injected with antigen every eight days for up to eight total injections with antigen. After the first injections, the others were prepared in the same way as the initial antigen, except that incomplete Freund's adjuvant was mixed with it prior to injection. The rabbits were bled, and serum was collected every fourteen days. This serum was checked for antibody titer by enzyme linked immunosorbent assay (ELISA). The ELISA assay was performed in microtiter wells as described by Rennard et al. (Anal Biochem. 104, 205-214, 1980). The antisera were checked for specificity by western immunoblot against laminin. Laminin was extracted and purified from the Engelbreth-Holm-Swarm tumor, which produces a basement membrane matrix, using methods previously described by Timpl et al., J. Biol. Chem. 254, 9933-9939, 1979; Fibronectin was purified from plasma using gelatin-Sepharose.

HT1080 cells (human fibrosarcoma) were maintained in Dulbecco's Modified Eagle's Medium (DMEM) supplemented with 0.06% glutamine, 1% sodium pyruvate, 1% penicillin-streptomycin, and 10% fetal calf serum. The HT1080 cells were grown to 80% confluency in Falcon T75 flasks. For adhesion assays, the cells were washed with phosphate buffered saline (PBS), pH 7.3, without Ca or Mg ions, followed by detachment with trypsin 0.025%, EDTA 0.025%. Trypsin was inactivated with medium containing 0.5% BSA. The cells were then pelleted by low speed centrifugation, resuspended in serum-free Eagle's Minimal Essential Medium (EMEM) containing 0.5% BSA (Miles Laboratories), and were then used immediately in the adhesion assay. Cell assays were performed in duplicate and the variability between duplicates was less than 5%. Chinese Hamster Ovary (CHO) cells were maintained in Dulbecco's Modified Eagle's Medium with 0.06% glutamine, 1% sodium pyruvate, 1% penicillin-streptomycin, (v/v from standard stock solution), 1 mM proline, and 10% fetal calf serum. The cells were grown and prepared for the adhesion assay the same way as the HT1080 cells. B16F10, murine melanoma cells, were maintained in DMEM supplemented with 0.06% glutamine, 1% penicillin-streptomycin, 1% non-essential amino acids, and 10% fetal calf serum. Cells were grown to 80% confluency in Falcon T75 flasks. For chemotaxis assays, the cells were washed once in PBS followed by a short trypsinization with 0.025% trypsin, 0.025% EDTA to detach the cells from the dish. The trypsin was inactivated by the addition of DMEM containing 10% fetal calf serum, followed by low speed centrifugation to pellet cells. The pellet was resuspended in serum-free DMEM containing 0.1% BSA. The cells were again pelleted by low speed centrifugation, and resuspended in serum free medium containing 0.1% BSA. These cells were immediately added to the upper compartment of the Boyden chamber.

Cell adhesion was assayed by adding various amounts of laminin and peptide to 35 mm tissue culture dishes and 1 ml of serum-free EMEM containing 0.5% BSA. HT1080 or CHO cells ($10^4$) were prepared as described above and added to each dish, followed by a one hour incubation at 37°C in 5% $CO_2$, 95% air. At the end of this period, plates were gently washed three times with PBS to remove unattached cells. Attached cells were trypsinized with 0.025% trypsin, 0.025% EDTA, and electronically counted. In some cases, bacteriologic petri dishes (35 mm) were coated with either laminin (5 micrograms/dish) or peptide (50 and 100 micrograms/dish) by diluting either in 1 ml of PBS and air drying the dish overnight. Prior to use, coated dishes were rinsed with serum free EMEM containing 0.5% BSA followed by the addition of cells in 1 ml of fresh EMEM with BSA. The remainder of the assay was carried out as described above. In some experiments, varying amounts of peptide were added to laminin-coated dishes (5 micrograms) containing 1 ml of serum-free EMEM with 0.5% BSA, prior to adding cells to see if the peptide could compete with laminin for cell attachment.

Another variation of this assay involved incubating different dilutions of antisera to the peptide albumin conjugates overnight at 4°C with laminin-coated substrates. One hour prior to adding the cells, the unbound antisera were removed, the plates were rinsed with PBS, and 1 ml of fresh serum-free EMEM with 0.5% BSA was added to each plate. The plates were then warmed to 37°C prior to adding the cells. Again the assay was carried out as previously described. The assay measured the ability of antisera to block laminin-mediated cell adhesion.

To measure cell migration, chemotaxis assays were carried out. Polycarbonate filters (8 microgram pore size; Neuroprobe) were coated with type IV collagen (10 micrograms/filter) and placed in a modified Boyden chamber. Cells (B16 F10 melanoma cells) were harvested and prepared as described above, and 3.5 x $10^5$ cells in 0.8 ml were placed in the upper compartment of the Boyden chamber. The lower compartment of the Boyden chamber contained the chemoattractants including various peptides (10-300 micrograms/ml), laminin (20 micrograms/ml), and fibronectin (20 micrograms/ml), in serum-free DMEM with 0.1% BSA (0.2ml). The chambers were then incubated for five hours at 37°C, 5% $CO_2$ and 95% air. The cells which had attached to the upper side of the filter were mechanically removed. The cells which had migrated to the lower side of the filter were fixed in methanol, and then stained with hematoxylin and eosin. Each sample was assayed in quadruplicate, and the cells in at least 5 microscopic fields per filter were counted.

The ability of the peptides to block migration to albumin was also tested. The preparation in terms of Boyden chamber, filters, and chemoattractants were as described above. The chemoattractants in the lower chamber were fibronectin (20 micrograms) or laminin (20 micrograms/ml). The cells were prepared as

EP 0 278 781 B1

described above and placed in the upper compartment of the Boyden chamber with the peptides being tested. The concentraton range of the peptides tested was 10 to 300 micrograms/ml.

It has been found that NG108-15 neuroblastoma x glioma cells attach and send out long neuronal processes in the presence of laminin. These cells were cultured to confluence in DMEM containing sodium bicarbonate, 0.06% glutamine, and supplemented with hypoxanthine ($1x10^{-4}$), aminopterin ($1x10^{-7}$), thymidine ($1.6x10^{-5}$ M), 1% penicillin-streptomycin, 0.01% gentamicin, and 5% fetal calf serum. These cells were then gently rinsed three times in 0.02M sodium phosphate, pH 7.4, scraped from the dish, and centrifuged at 1000 rpm for five minutes. The cell pellet was then sonicated in 0.01 M Tris-HCl, pH 7.4, containing 1% CHAPS (3[3-chloromidopropyl]) dimethylammonio]-1-propane and 0.002 M phenylmethylsulfonyl fluoride, and extracted for two hours at 4°C. After centrifugation at 10,000 rpm for 20 minutes, the supernatant fluid was applied to a laminin affinity column. Proteins were chromatographed on the laminin affinity columns in a manner analogous to that used to isolate the laminin receptor as described by Lesot et al. (EMBO J 2 861-865, 1983). The sample was circulated on two columns run in parallel and equilibrated in 0.01 M Tris-HCl, pH 7.4, containing 0.1% CHAPS for two hours. After the unbound material was collected, the columns were washed further with the column buffer. Peptides designated as peptide 11 or peptide 12 at 1 mg/ml in the column buffer were added to the columns and allowed to remain in contact with the resin for 10 minutes. The eluted materials were then washed from the column. The starting material, unbound material, and peptide 11 or 12-eluted material were dialyzed against water and lyophilized. Gel electrophoresis in 7.5% polyacrylamide was carried out on aliquots of the samples.

In an analogous study, affinity columns composed of peptide 11 and peptide 12 were prepared and exposed to the NG108-15 cell extracts. The columns were run in a similar manner, and the bound material was eluted with 1M NaCl and 0.1% CHAPS followed by 0.1M glycine, pH 2. The starting material and unbound and bound materials were dialyzed, lyophilized, electrophoresced, and blotted onto nitrocellulose. The nitrocellulose filters were then exposed to anti-67 Kd laminin receptors antibody using established procedures.

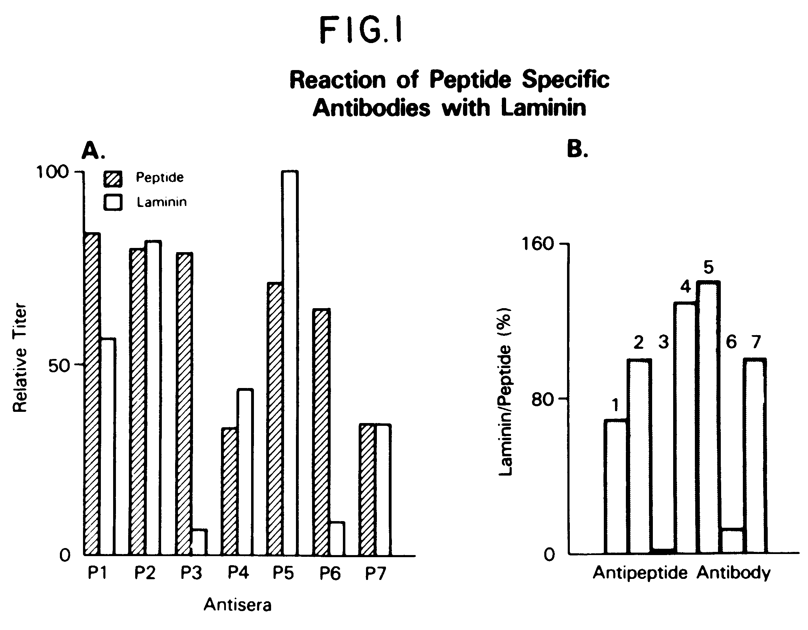

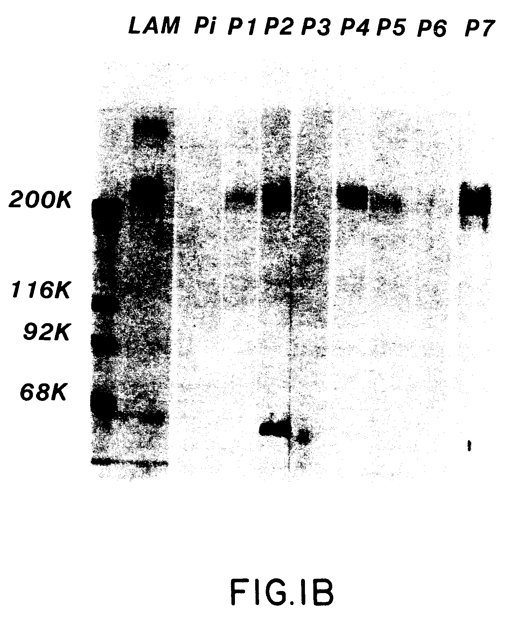

Peptides (20 mers) corresponding to each structural domain in the B1 chain of laminin were synthesized as well as conjugated to albumin. The sequences chosen for investigation represent highly hydrophilic regions which were expected to be antigenic, allowing peptide-specific antibodies to be prepared to the conjugates in rabbits. Very specific antibody titers were obtained from immunized animals which were specific to the particular conjugate, and the antibodies could be purified by affinity chromatography on peptide-Sepharose. The antibodies to peptides 1, 2, 4, 5 and 7 showed a good reaction with native laminin in ELISA assays, and these antibodies reacted specifically with the B chain of laminin in Western blots. Antibody to peptides 3 and 6 reacted well with their corresponding peptides but had little or not cross reaction with laminin. The results of these assays are shown in Figure 1A and B.

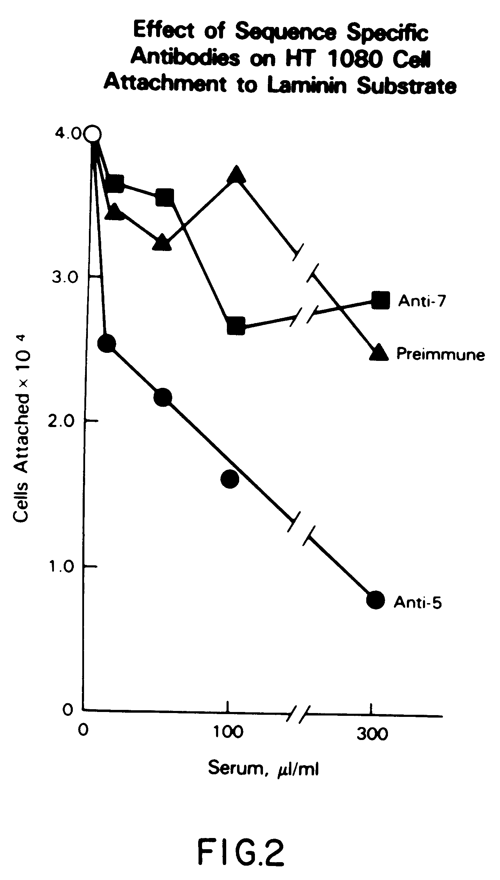

Each peptide was absorbed to plastic and assayed for its ability to promote the attachment of HT1080 and CHO cells. No significant cell attachment activity was found in any of these peptides. The antisera to the peptides were then also tested for their ability to inhibit epithelial cell attachment to a laminin substrate. Only antibodies to peptide 5 conjugate inhibited cell attachment. The results of this assay are in Figure 2.

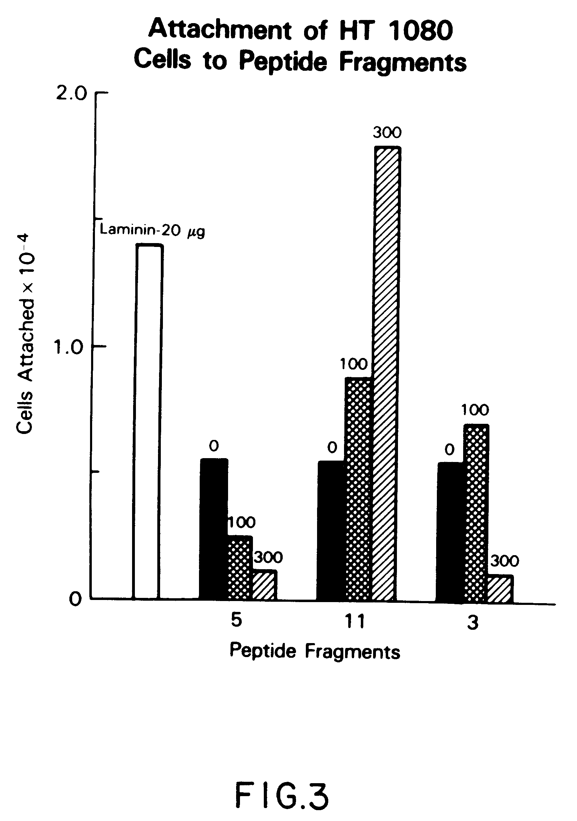

Since the corresponding peptide fragment did not promote cell attachment, it was concluded that the actual attachment site occurred in the vicinity of this sequence, and that the active site was blocked by the antibody to peptide 5 for steric reasons. Two peptides, a 9 mer, peptide 11, and a 10 mer, peptide 12, were chosen for synthesis and study based in part on sequence homology to EGF and on their proximity to peptide 5. Peptide 11 was found to stimulate cell attachment in a dose-dependent fashion to the levlts obtained with laminin itself. None of the other peptides demonstrated attachment activity when coated on a dish. The results of these assays are shown in Figure 3.

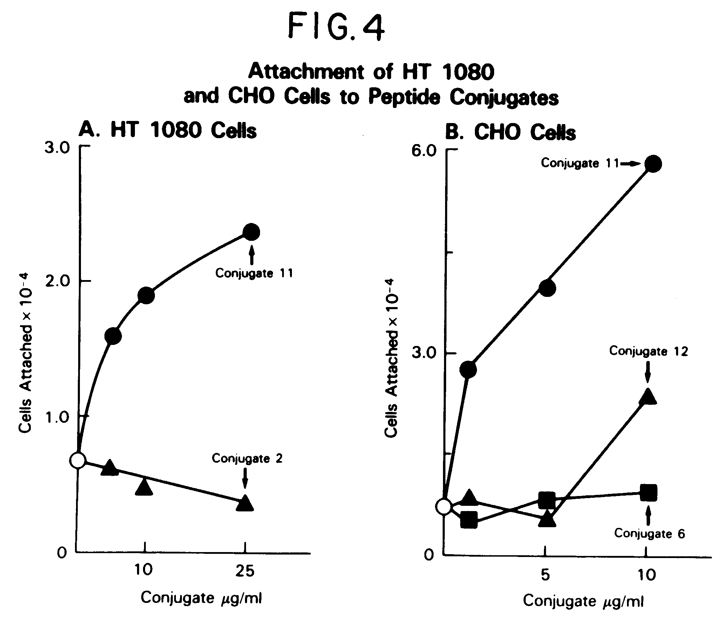

Peptide albumin conjugates were also tested in the cell attachment assays because it was thought that albumin conjugates might increase the availability of the peptide and thus the potency of the peptides in this assay. Peptide 11 albumin conjugate supported the attachment of HT1080 and CHO cells when used at levels of 25 micrograms/ml, while the other peptide conjugates were without activity in this assay. The results of this assay are shown in Figure 4. It was estimated by direct immunoassay that these conjugates represented about 20% peptide, indicating that they show on a molar basis 0.5-1.0% of the activity of laminin itself in cell attachment.

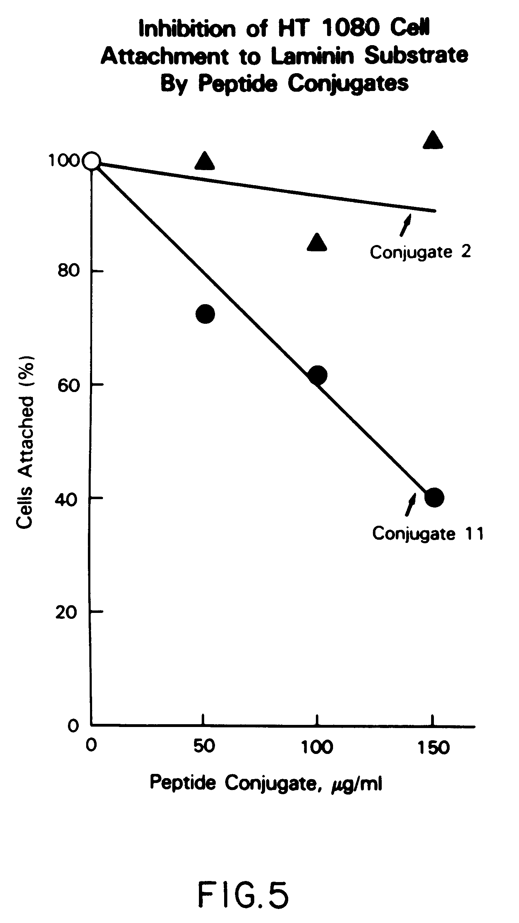

Since it was possible that a completely independent receptor on the cells was binding to peptide II rather than the laminin receptor, the ability of these peptides to inhibit attachment to laminin substrates when peptides were added to the coated dishes along with the cells was assessed. Only peptide 11, and none of the others, suppressed the attachment of the cells to laminin. These results indicate that the sequence of amino acids in peptide 11 encompasses the sequence of amino acids involved in attachment to laminin itself. The results of these assay are shown in Figure 5.

6

Since laminin is known to promote directed cell migration (chemotaxis), the peptides obtained therefrom were tested for chemotactic activity using B16F10 cells, a murine melanoma cell line which is attracted to both laminin and fibronectin. Peptides 1-7 and peptide 12 showed no significant chemotactic activity toward the melanoma cells when placed in the lower compartment of the Boyden chamber, nor ability to inhibit chemotaxis when placed together with the cells in the upper compartment. In contrast, peptide 11, the active portion of which is Tyr-Ile-Gly-Ser-Arg, was found to be a chemoattractant for the melanoma cells, showing about 32% of the maximum response observed with optimal levels of laminin. The results are shown in Table I.

Table I

EFFECT OF SYNTHETIC PEPTIDES

ON CELL MIGRATION

A. Stimulation

| Chemoattractant | Cells Migrated | Activity (%) |
|---|---|---|
| Laminin | $41.6 \pm 2.5$[a] | 100 |
| Peptide 11 | $13.5 \pm 1.5$ | 32 |
| Peptide 2 | $1.8 \pm 0.5$ | 4 |
| No additions | $3.0 \pm 0.7$ | 7 |

B. Competition

| Compound | | Cells Migrated | Activity (%) |
|---|---|---|---|
| Lower | Upper | | |
| Laminin | None | $4.16 \pm 0.7$ | 100 |
| Laminin | Peptide 11 | $9.7 \pm 0.6$ | 23 |
| Laminin | Peptide 2 | $38.7 \pm 0.3$ | 93 |
| Fibronectin | None | $16.3 \pm 0.6$ | 100 |
| Fibronectin | Peptide 11 | $12.3 \pm 0.8$ | 75 |
| Fibronectin | Peptide 2 | $15.7 \pm 0.4$ | 96% |

[a]Data are presented as $\pm$ SEM

7

The fact that this was a true chemotactic response was confirmed by the so-called "checker board" assay, wherein the levels of attractants are systematically varied in the upper and lower compartments of the Boyden chamber.

To establish that the cells recognized similar epitopes on laminin, the ability of the peptides to alter the chemotactic response of the B16 F10 cells to laminin was tested. Only peptide 11 inhibited the chemotactic response of the cells to laminin. Little or no inhibition of chemotaxis to fibronectin was observed, indicating that peptide 11 was showing a specific competition with laminin. These results are shown in Table IB.

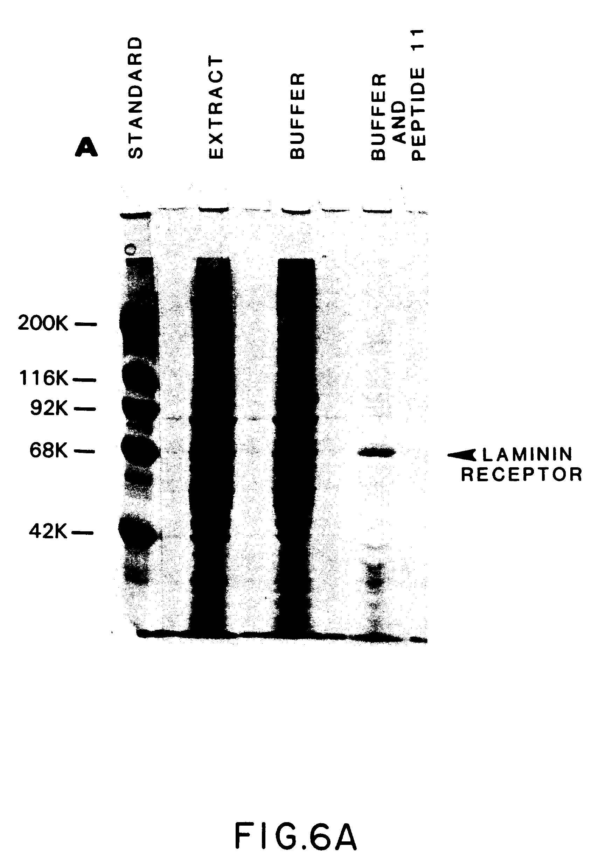

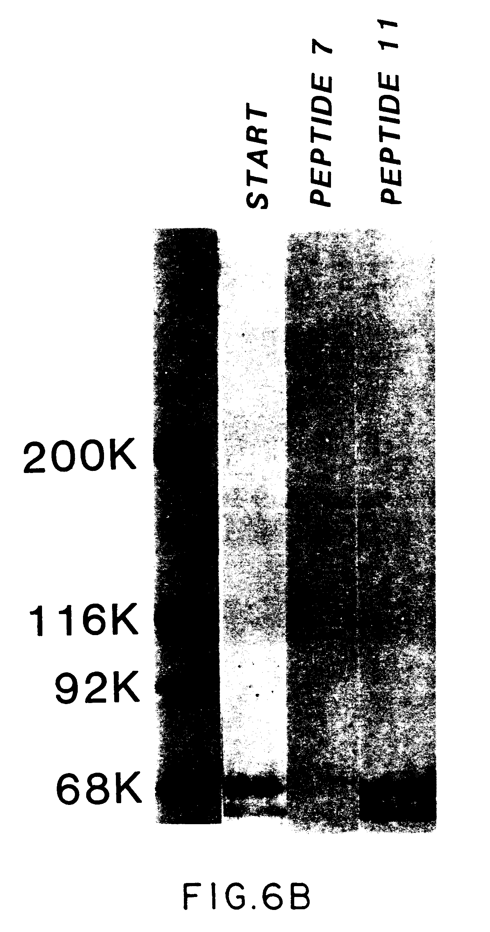

Since cells bind to laminin through a specific receptor (Mr = 67,000), it was determined whether peptide 11 was able to elute the laminin receptor from a laminin affinity column. A cell extract from NG-108 cells, a murine neuroblastoma cell line which attaches to laminin, was applied to a laminin affinity column and the unbound material was removed with several washes. Subsequently, the column buffer plus peptide 11 (1 mg/ml) was used as eluent, and the proteins in the starting material, unbound material, and peptide eluted material were resolved by electrophoresis. Buffer containing peptide 11 eluted a single major protein (Mr = 67,000) as shown in Figure 6, which reacted with authentic antibody to the laminin receptor. Comparable studies with peptide 12 showed no ability to elute the receptor from laminin. The results of this assay are shown in Fig. 6A and 6B.

Peptide 11, Cys-Asp-Pro-Gly-Tyr-Ile-Gly-Ser-Arg, (CDPGYIGSR) and its amide form have been tested in vivo for their ability in vitro and in vivo to inhibit tumor cell invasion and metastases, respectively. In both assays, peptide 11 inhibits tumor cell invasion and metastasis, and the amide form of peptide 11 is even more potent in inhibiting tumor cell invasion and metastasis.

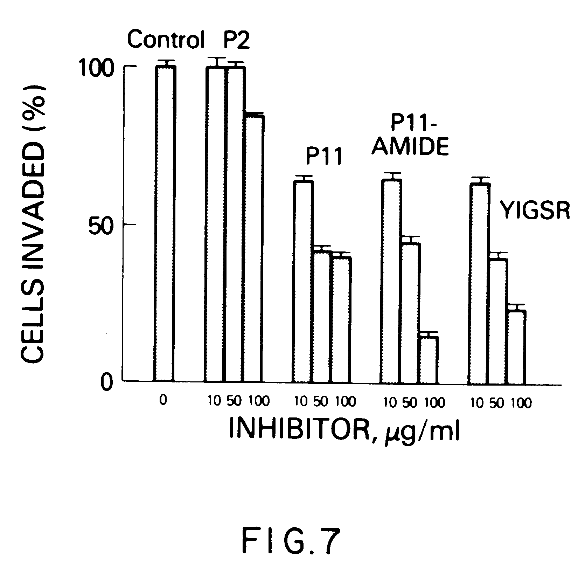

Various peptides from the B1 chain of laminin were tested for their ability to inhibit B16 F10 melanoma cell invasion of a basement membrane in vitro. Only peptide 11 and its amide form were able to inhibit invasion, while peptides 1-7 and 12 were inactive. Peptide 11 was able to inhibit, by approximately 50%, at 50 and 100 micrograms/ml, whereas the peptide 11 amide was more active and able to inhibit invasion by approximately 80% at 100 micrograms/ml. The results of this assay are shown in Fig. 7.

Blind well chemotaxis chambers with 13 mm diameter filters were used for the assay. Polyvinylpyrrolidone-free polycarbonate filters, 8 micron pore size (Nucleopore, California) were coated with basement membrane matrigen (50 micrograms/filter) and placed into Boyden chambers. B16 F10 melanoma cells (3 x 10$^5$), suspended in DMEM containing 0.1% BSA, were added to the upper chamber.

Conditioned medium was obtained by incubating fibroblasts for 24 hours in serum-free medium in the presence of ascorbate. This medium was used as a source of chemoattractants and placed in the lower compartment of the Boyden chambers. Assays were conducted at 37°C. in 5% $CO_2$. At the end of the incubation, the cells on the upper surface of the filter were removed mechanically. The filters were fixed in methanol and were stained with hematoxylin/eosin. Cells from various areas of the lower surface were counted and each assay was performed in triplicate.

Peptide 11 and its amide form were tested in vivo at both 100 micrograms and 1 milligram per animal for their ability to inhibit lung metastases formation. At 100 micrograms, peptide 11 was able to slightly inhibit the formation of lung metastases two weeks after injection into the mice. At 1 milligram, peptide 11 significantly inhibited lung metastases. Peptide 11 amide was more active than peptide 11 at both 100 micrograms and 1 milligram in blocking lung melanoma formation. These data are shown in Tables IIA and IIB.

To test for inhibition of metastases, peptide 11 and peptide 11 amide at 100 micrograms and 1 mg were preincubated with B16 F10 melanoma cells (5 x 10$^5$) in a final volume of 0.2 ml. The cells and peptide were injected via the tail vein using standard procedures into C57B1/6 mice. Eight mice were injected for each test concentration. Control mice received only the cells. After two weeks, the mice were sacrificed and the lungs were removed and photographed. Sections were made and the number of metastases was quantified. The results are shown in Tables IIA and IIB, below.

## TABLE IIA

### NUMBER OF PULMONARY METASTASES
### FROM 5 X $10^5$ MELANOMA CELLS

#### Two Weeks After Injection into the Mice

| | Mean Mean Pulmonary Metastases | % Inhibition |
|---|---|---|
| Control | 267.8 | 0 |
| Peptide 11, 100 $\mu$grams | 230.0 | 14.1 |
| Peptide 11, 1 mg | 69.5 | 74.1 |
| Peptide 11 amide, 100 $\mu$grams | 151.9 | 43.3 |
| Peptide 11 amide, 1 mg | 4.9 | 98.4 |

#### EFFECT OF SYNTHETIC PEPTIDES ON IN VIVO METASTASES

| | O | 50 | 100 | 500 $\mu$g peptide |
|---|---|---|---|---|
| YIGSR-NH$_2$ | < 200 | 70 | 45 | 5 |
| cyclic YIGSR | < 200 | 32 | 20 | 3 |
| PDSGR-NH$_2$ | < 200 | 65 | 60 | 24 |

Data are expressed as mean number of pulmonary metastases. Eight mice were used for each group.

The peptides were tested for their possible toxic effects on cells. Based on trypan blue exclusion and cell viability, the peptides were not found to have any toxic effects.

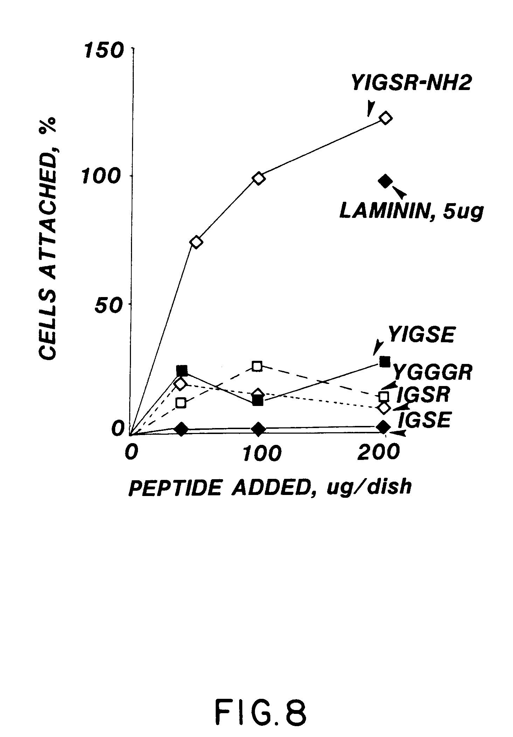

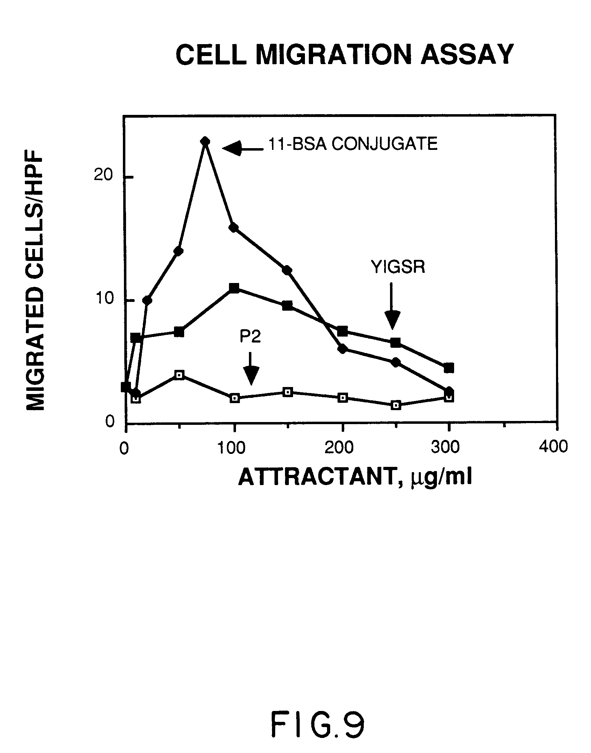

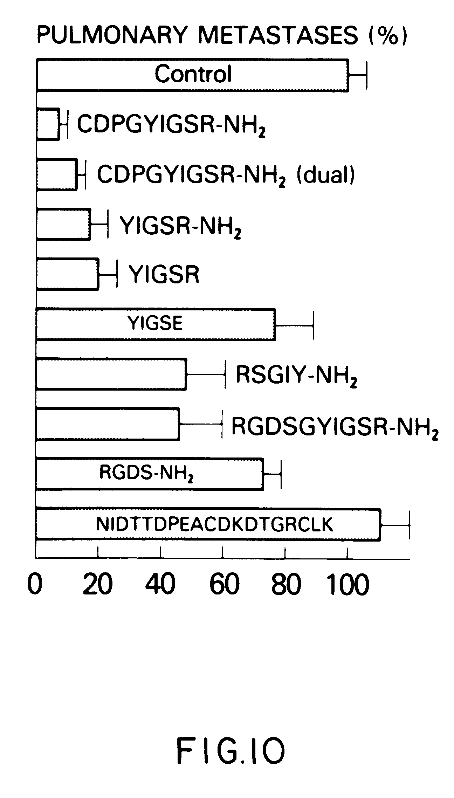

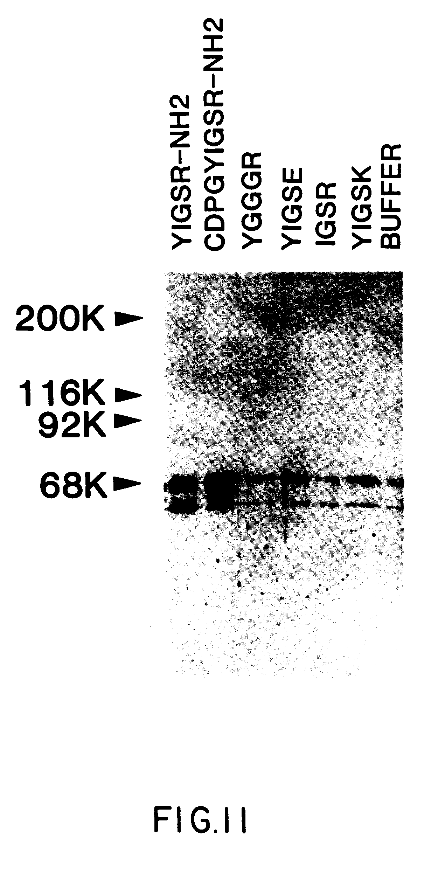

Table III shows a series of synthetic peptides having laminin-like activities. The pentapeptide YIGSR, i.e., Tyr-Ile-Gly-Ser-Arg, is the smallest active peptide which is able to promote cell attachment, cell migration and receptor elution, and inhibit tumor cell metastases. The results are shown in Table III and Figures 8, 9, 10 and 11.

TABLE III

| Synthetic Peptides Tested for Laminin-like Activities | Relative[*] Activities |
|---|---|
| Laminin | + + + + |
| 1. C D P G Y I G S R | + + + |
| 2. C D P G Y I G S R - $NH_2$ | + + + + |
| 3. C D P G Y I G S R - 1,2 cyclohexanedione | o |
| 4.    D P G Y I G S R | + + + |
| 5.       G Y I G S R | + + + |
| 6.          Y I G S R - $NH_2$ | + + + + |
| 7.          Y I G S R | + + + |
| 8.          Y I G S E | + |
| 9.          Y G G G R | + |
| 10.          Y I G S K | + |
| 11.          I G S R | + |
| 12.          I G S E | o |
| 13.          R S G I Y - $NH_2$ | + + |

[*] Attachment of epithelial cells - all were tested
Migration of tumor cells - all but 3, 4, 8, 9, 10, 11, 12, 13 were tested
Laminin receptor binding - all but 3 and 13 were tested
Antimetastatic activity - all but 3, 4, 5, 9, 10, 11, 12 were tested

As demonstrated above, peptide 11, its amide form, and the active portion tyr-ile-gly-ser-arg, clearly have antimetastatic activity. The peptides can be conjugated to human or homologous albumin, which allows lower amounts to be used, as the albumin conjugate is maintained in the circulation longer than the unconjugated peptide. Additionally, the peptide could be polymerized or cyclized as the polymerized or cyclized peptide would be less likely to be cleared from the circulation and would be effective at a lower dose as an anti-metastatic agent (Table IIB).

The peptides of the present invention can be used as a carrier to target drugs to metastatic tumor cells. Because of this ability to target tumor cells, the peptide can also be conjugated to an anti-cancer agent for therapy.

The peptides of the present invention can be used as a cell-attachment protein to provide substrata to which cells will attach by treating a hydrophobic surface, such as untreated synthetic plastic resin material such as nitrocellulose, or comparable material, with the polypeptide. A similar substratum for cell attachment can be generated by coupling the polypeptide covalently to a solid support, such as glass or a synthetic plastic resin or a long chain polysaccharide, such as agarose, containing a reactive group that can bind the polypeptide. This latter approach can be effected by coupling the peptide to cyanogen bromide-activated agarose beads (sold under the trademark Sepharose by Pharmacia Fine Chemicals, Uppsala, Sweden) sterilizing the beads by autoclaving, and thereafter showing that the peptide coating induces attachment of cells to the beads in a concentration greater than can be obtained by passive absorption.

It has also been found that the peptides of the present invention, i.e., those peptides containing the YIGSR (tyrosine-isoleucine-glycine-serine-arginine) sequence, can alter the formation of capillary structures by endothelia cells, and to inhibit angiogenesis (vascularization). The angiogenesis inhibition was demonstrated in a commonly used assay using chick chorioallantoic membrane.

Human skin endothelial cells plated onto matrigel, a reconstituted basement membrane which is the subject of U.S. Patent Application Serial No. 0161867, filed May 27, 1986, and incorporated herein by reference, rapidly aligned and formed capillary-like structures. The cells showed a very different behavior on plastic- or collagen-coated surfaces, forming a monolayer of single cells. More importantly, the addition of YIGSR amide (tyrosine-isoleucine-glycine-serine-arginine-amide) to the media of cells plated on matrigel, or the inclusion of this peptide (YIGSR amide) within the gel, inhibited the endothelial cells from forming capillary-like structures.

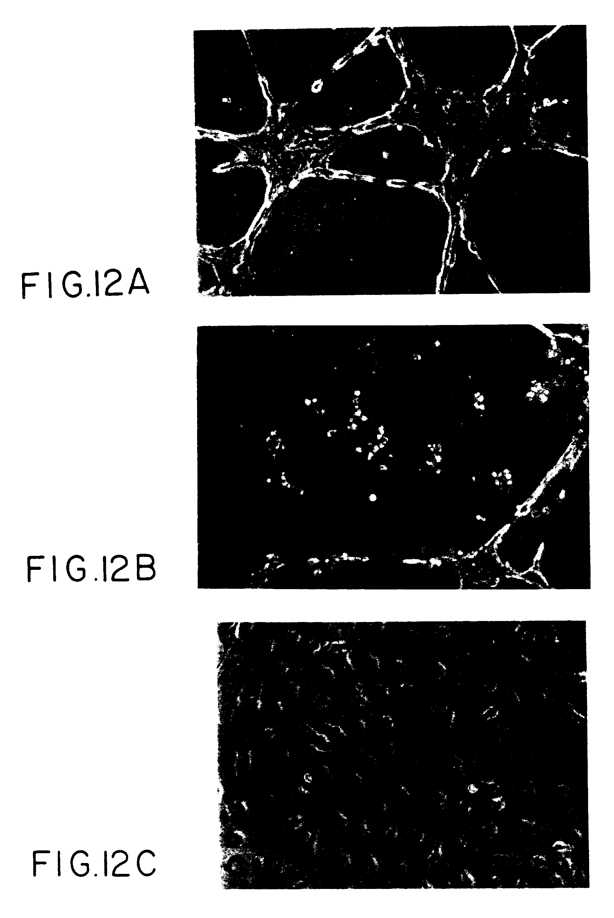

This effect is the subject of Figure 12, which shows the effect of YIGSR-amide on capillary endothelial cell tube formation. In this Figure, A shows endothelial cells which, when plated on reconstituted basement membrane, form tubule-like structures within 18 hours. In B, the formation of the tubules is blocked by 0.5 mg/ml of YIGSR-amide. In C, the cells form a monolayer on type I collagen.

Additionally, the YIGSR amide was tested in the chorioallantoic membrane assay in chick embryos. In these studies, the YIGSR amide placed within the tissue inhibited blood vessels from forming in the region of the peptide.

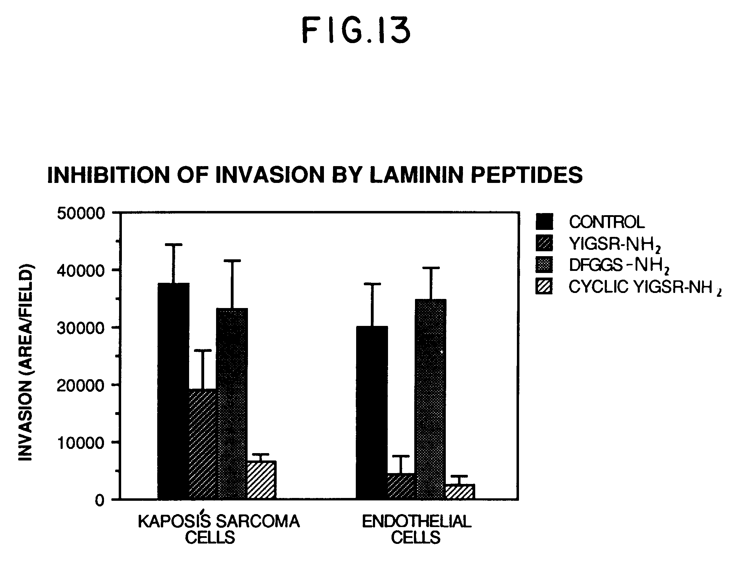

The peptide YIGSR-amide and a cyclic peptide formed from YIGSR were tested on the invasion of endothelial cells through a reconstituted basement membrane, using the test described in Cancer Res. 47: 3239-3245, 1987. In this assay, cells must adhere, degrade, and migrate through the matrix to be considered invasive. As shown in Figure 13, both peptides were potent inhibitors of endothelial cell invasion through basement membrane. This process is also necessary for angiogenesis in vivo. Thus, the YIGSR peptides of the present invention are capable of blocking angiogenesis (neovascularization), indicating that these peptides have a variety of applications in inhibiting growth to preventing an excess of blood vessels in tissues due to inflammation or to other pathological conditions such as Kaposi sarcoma.

The peptides of the present invention can also be used for preparing surfaces for optimal cell culture, derivatization of various prosthetic materials to promote bonding with surrounding tissues, providing for the increased internalization of molecules such as toxins, drugs, hormones, or the like by the enhancement of phagocytosis, and the development of ways of manipulating cellular adhesion mechanisms in diseases such as cancer metastasis and platelet aggregation.

It is expected that such substrata will be useful in cell cultures where it is desirable to ensure proper attachment of the cells. Attachment proteins such as laminin, have been shown to be important for the growth of many types of cells in vitro. Chemically defined media are often supplemented by attachment proteins (cf. Barnes and Sato, Cell 22:649-655, 1980). Coating of the culture substratum with the cell-attachment peptide would obviate the use of laminin in the medium, thus providing better defined conditions for the culture, as well as better reproducibility. An example of the commercial use of cell attachment surfaces is the Cytodex particles manufactured by Pharmacia wherein the particles are coated with gelatin, making it possible to grow the same number of adherent cells in a much smaller volume of media than would be possible in dishes. The activity of these beads is, however, dependent upon the use of laminin in the growth medium in most cases. The cell-attachment peptide of the present invention should provide a chemically defined coating for such purposes.

Medical devices can be designed which make use of such substrata to attract cells to the surface in vivo or even to promote the growing of a desired cell type on a particular surface prior to grafting. An example of this is endothelial cell growth on a prosthetic blood vessel or vascular graft, which is generally woven or knitted from polyester fiber, particularly Dacron fiber (a polyethylene terephthalate). Because most types of cells are attracted to laminin and to the peptides of the present invention, the peptides of the present invention are useful in coating a patch graft or the like for aiding wound closure and healing following an accident or surgery. The peptides of the present invention can also be used in coating surfaces of a prosthetic device which is intended to serve as a temporary or semipermanent entry into the body, e.g., into a blood vessel or into the peritoneal cavity, sometimes referred to as a percutaneous device. In such cases, it may be advantageous to couple the peptide to a biological molecule, such as collagen, a glycosaminoglycan, or a proteoglycan.

The peptides of the present invention can be administered in amounts ranging from about 10 micrograms to about 20 milligrams per kilogram of body weight.

The peptides of the present invention may be used in the form of a liquid, such as eye drops or lotions, or a salve or gel which may be applied to promote cell attachment, or in any other convenient form. Accordingly, the peptides may be contained in any pharmaceutically acceptable carrier which is appropriate for the delivery means intended. One manifestation of the cell attachment activity of the peptides of the present invention is their chemotactic activity.

## Claims

1. Peptides characterised in having laminin-like activity and being selected from:
   (1) tyrosine-isoleucine-glycine-serine-arginine;

11

(2) cysteine-aspartate-proline-glycine-tyrosine-isoleucine-glycine-serine-arginine;

(3) aspartate-proline-glycine-tyrosine-isoleucine-glycine-serine-arginine;

(4) glycine-tyrosine-isoleucine-glycine-serine-arginine;

and derivatives thereof selected from amides, conjugates with proteins, cyclized peptides, and polymerized peptides.

2. The peptide of claim 1 which is tyrosine-isoleucine-glycine-serine arginine.

3. The peptide of claim 1 which is cysteine-aspartate-proline-glycine-tryosine-isoleucine-serine-arginine.

4. The peptide of claim 1 which is aspartate-proline-glycine-tyrosine-isoleucine-glycine-serine-arginine.

5. The peptide of claim 1 which is glycine-tyrosine-isoleucine-glycine-serine-arginine.

6. A peptide of any of claims 1 to 5 attached to the surface of a synthetic resin fibre.

7. A peptide of any of claims 1 to 5 attached to the surface of a percutaneous device.

8. A peptide of any of claims 1 to 5 coupled to collagen.

9. A peptide of any of claims 1 to 5 in the form of a lotion, salve, gel, colloid, or powder.

10. A peptide characterised by laminin-like activity which is the amide form of proline-aspartate-serine-glycine-arginine.

11. An anti-metastatic agent for metastatic tumor cells, characterised in that it comprises a peptide of any of claims 1 to 10 or its amide form in a suitable carrier for an anti-metastatic agent.

12. An agent for inhibiting the formulation of lung metastases, characterised in that it comprises a lung-metastasis-inhibiting effective amount of a peptide of claims 1 to 10 or its amide form.

13. An agent for promoting the migration of epithelial cells in a wound such as in the cornea and the like, characterised in that it comprises a migration-promoting amount of a peptide of any of claims 1 to 9 or its amide form.

14. An agent for inhibiting the formation of capillary-like structures by endothelial cells, characterised in that it comprises a capillary-like structure formation inhibiting effective amount of a peptide of any of claims 1 to 9 or its amide form.

15. An agent for blocking new blood vessel formation, characterised in that it comprises a new blood vessel formation blocking effective amount of a peptide of any of claims 1 to 9 in a suitable carrier.

16. An agent for blocking blood vessel growth in tissues, characterised in that it comprises a blood vessel growth blocking effective amount of a peptide of any of claims 1 to 9 in a suitable carrier.

17. An agent for treating Kaposi's sarcoma, characterised in that it comprises a Kaposi's sarcoma treating effective amount of the peptide of any of claims 1 to 10.

18. An agent for promoting increased adhesion of epithelial or endothelial cells to vascular prostheses and other artificial organs; characterised in that it comprises a peptide of any of claims 1 to 9 or its amide form.

19. An anti-adhesion factor for laminin-responsive epithelial cells, characterised in that it comprises a compound selected from a peptide of any of claims 1 to 9 and its amide form and its BSA conjugate.

20. An anti-adhesion factor for laminin-responsive epithelial cells, characterised in that it comprises an antibody against a conjugate of a peptide of any one of claims 1 to 9, and BSA or another component.

**21.** A migration promoting factor for epithelial cells, characterised in that it comprises a compound selected from a peptide of any of claims 1 to 9 and its amide form and its BSA conjugate.

**22.** A migration inhibition factor for laminin responsive epithelial cells, characterised in that it comprises a compound selected from a peptide of any of claims 1 to 9 and its amide form and its BSA conjugate.

**23.** A substrate active in promoting epithelial, endothelial or neural cell attachment, characterised in that it comprises a compound selected from a peptide of any of claims 1 to 9 its amide form and its BSA conjugate.

**24.** A surface treated substrate, characterised in that it comprises a peptide of any of claims 1 to 9, attached to the surface of a solid substrate to ensure that cells will attach to said substrate.

**25.** The surface treated substrate of claim 24, wherein the substrate is selected from glass, a synthetic resin, and a long-chain polysaccharide.

**26.** The surface treated substrate of claim 24, wherein the substrate is selected from nitrocellulose and polyester.

**27.** The surface treated substrate of claim 24, wherein the substrate is agarose.

**28.** A prosthetic device characterised by having a biologically active surface which exhibits cell attachment activity, said surface having linked thereto a peptide of any of claims 1 to 9.

**29.** The prosthetic device of claim 28, wherein said surface constitutes a portion of a vascular graft.

**30.** The prosthetic device of claim 28, wherein said surface is made of a synthetic resin fibre.

**31.** The prosthetic device of claim 28, wherein said surface comprises a portion of a percutaneous device.

**32.** A method for promoting increased adhesion of epithelial or endothelial cells to vascular prostheses and other artificial organs, characterised in that it comprises in vitro coating the prostheses or organs with a compound selected from a peptide of any of claims 1 to 9 or its amide form.

**33.** A method for isolating the laminin cell surface receptor from detergent extracts of cells or of cell membranes bound to a laminin affinity column, characterised in that it comprises adding to said cells or cell membranes a compound selected from a peptide of any of claims 1 to 9 and its amide form.

**34.** A carrier to target metastatic tumor cells characterised in that it comprises a peptide of any of claims 1 to 10 or its amide form.

**35.** A laminin B chain specific antibody, characterised in that it comprises an antibody against the BSA conjugate of a peptide of any of claims 1 to 10.

**36.** A composition characterised in that it promotes the attachment of cells to a substrate when immobilized on said substrate and comprises a peptide of any of claims 1 to 9.

**Patentansprüche**

**1.** Peptide, dadurch gekennzeichnet, daß sie Laminin-ähnliche Aktivität haben und ausgewählt sind aus:
    (1) Tyrosin-Isoleucin-Glycin-Serin-Arginin;
    (2) Cystein-Aspartat-Prolin-Glycin-Tyrosin-Isoleucin-Glycin-Serin-Arginin;
    (3) Aspartat-Prolin-Glycin-Tyrosin-Isoleucin-Glycin-Serin-Arginin;
    (4) Glycin-Tyrosin-Isoleucin-Glycin-Serin-Arginin;
und Derivaten hiervon, die aus Amiden, Konjugaten mit Proteinen, zyklisierten Peptiden und polymerisierten Peptiden ausgewählt sind.

**2.** Peptid nach Anspruch 1, das Tyrosin-Isoleucin-Glycin-Serin-Arginin ist.

EP 0 278 781 B1

**3.** Peptid nach Anspruch 1, das Cystein-Aspartat-Prolin-Glycin-Tyrosin-Isoleucin-Serin-Arginin ist.

**4.** Peptid nach Anspruch 1, das Aspartat-Prolin-Glycin-Tyrosin-Isoleucin-Glycin-Serin-Arginin ist.

**5.** Peptid nach Anspruch 1, das Glycin-Tyrosin-Isoleucin-Glycin-Serin-Arginin ist.

**6.** Peptid nach einem der Ansprüche 1 bis 5, das an die Oberfläche einer Kunstharzfaser angelagert ist.

**7.** Peptid nach einem der Ansprüche 1 bis 5, das an die Oberfläche eines perkutanen Geräts angelagert ist.

**8.** Peptid nach einem der Ansprüche 1 bis 5, das mit Collagen gekoppelt ist.

**9.** Peptid nach einem der Ansprüche 1 bis 5 in Form einer Lotion, einer Salbe, eines Gels, eines Kolloids oder eines Pulvers.

**10.** Peptid, gekennzeichnet durch Laminin-artige Aktivität, das in der Amid-Form von Prolin-Aspartat-Serin-Glycin-Arginin vorliegt.

**11.** Anti-metastatisches Mittel für metastatische Tumorzellen, dadurch gekennzeichnet, daß es ein Peptid nach einem der Ansprüche 1 bis 10 oder seine Amid-Form in einem geeigneten Träger für ein anti-metastatisches Mittel enthält.

**12.** Mittel zur Hemmung der Bildung von Lungenmetastasen, dadurch gekennzeichnet, daß es eine Lungenmetastasenhemmende wirksame Menge eines Peptids nach den Ansprüchen 1 bis 10 oder seine Amid-Form enthält.

**13.** Mittel zur Förderung des Wanderns von Epithelzellen in einer Wunde, beispielsweise in der Hornhaut und dgl., dadurch gekennzeichnet, daß es eine wanderungsfördernde Menge eines Peptids eines der Ansprüche 1 bis 9 oder seine Amid-Form enthält.

**14.** Mittel zur Hemmung der Bildung kapillarähnlicher Strukturen durch Endothelzellen, dadurch gekennzeichnet, daß es eine zur Hemmung der Bildung einer kapillarähnlichen Struktur wirksame Menge eines Peptids nach einem der Ansprüche 1 bis 9 oder seine Amid-Form enthält.

**15.** Mittel zur Blockierung der Bildung neuer Blutgefäße, dadurch gekennzeichnet, daß es eine zur Blockierung der Bildung neuer Blutgefäße wirksame Menge eines Peptids nach einem der Ansprüche 1 bis 9 in einem geeigneten Träger enthält.

**16.** Mittel zum Blockieren des Blutgefäßwachstums in Geweben, dadurch gekennzeichnet, daß es eine zum Blockieren des Blutgefäßwachstums wirksame Menge eines Peptids nach einem der Ansprüche 1 bis 9 in einem geeigneten Träger enthält.

**17.** Mittel zur Behandlung des Kaposi-Sarkoms, dadurch gekennzeichnet, daß es eine zur Behandlung des Kaposi-Sarkoms wirksame Menge des Peptids nach einem der Ansprüche 1 bis 10 enthält.

**18.** Mittel zur Förderung einer gesteigerten Adhäsion von Epithel oder Endothelzellen an Vaskularprothesen und anderen künstlichen Organen, dadurch gekennzeichnet, daß es ein Peptid nach einem der Ansprüche 1 bis 9 oder seine Amid-Form enthält.

**19.** Anti-Adhäsions-Faktor für auf Laminin reagierende Epithelzellen, dadurch gekennzeichnet, daß es eine aus einem Peptid nach einem der Ansprüche 1 bis 9 und seiner Amid-Form sowie seinem BSA-Konjugat ausgewählte Verbindung enthält.

**20.** Anti-Adhäsions-Faktor für auf Laminin reagierende Epithelzellen, dadurch gekennzeichnet, daß es einen Antikörper gegen ein Konjugat eines Peptids nach einem der Ansprüche 1 bis 9 und BSA oder eine andere Komponente enthält.

14

**21.** Wanderungsförderungsfaktor für Epithelzellen, dadurch gekennzeichnet, daß er eine aus einem Peptid nach einem der Ansprüche 1 bis 9 und seiner Amid-Form und seinem BSA-Konjugat ausgewählte Verbindung enthält.

**22.** Wanderungshemmungsfaktor für auf Laminin reagierende Epithelzellen, dadurch gekennzeichnet, daß er eine aus einem Peptid nach einem der Ansprüche 1 bis 9 und seiner Amid-Form und seinem BSA-Konjugat ausgewählte Verbindung enthält.

**23.** Für Epithel-, Endothel- oder Nervenzellenanhaftung förderungsaktives Substrat, dadurch gekennzeichnet, daß es eine aus einem Peptid nach einem der Ansprüche 1 bis 9, seiner Amid-Form und seines BSA-Konjugats ausgewählte Verbindung enthält.

**24.** Oberflächenbehandeltes Substrat, dadurch gekennzeichnet, daß es ein Peptid nach einem der Ansprüche 1 bis 9 enthält, das an die Oberfläche eines festen Substrats angelagert ist, um das Anlagern von Zellen an das Substrat sicherzustellen.

**25.** Oberflächenbehandeltes Substrat nach Anspruch 24, wobei das Substrat aus Glas, einem Kunstharz und einem langkettigen Polysaccherid ausgewählt ist.

**26.** Oberflächenbehandeltes Substrat nach Anspruch 24, wobei das Substrat aus Nitrozellulose und Polyester ausgewählt ist.

**27.** Oberflächenbehandeltes Substrat nach Anspruch 24, wobei das Substrat Agarose ist.

**28.** Prothese, gekennzeichnet durch eine biologisch aktive Oberfläche, die Zellenanhaftungsaktivität zeigt, wobei die Oberfläche ein daran gekoppeltes Peptid nach einem der Ansprüche 1 bis 9 aufweist.

**29.** Prothese nach Anspruch 28, wobei die Oberfläche einen Teil eines Vaskularimplantats bildet.

**30.** Prothese nach Anspruch 28, wobei die Oberfläche aus einer Kunstharzfaser hergestellt ist.

**31.** Prothese nach Anspruch 28, wobei die Oberfläche einen Teil eines perkutanen Geräts darstellt.

**32.** Verfahren zur Förderung gesteigerter Adhäsion von Epithel- oder Endothelzellen an Vaskularprothesen und anderen künstlichen Organen, dadurch gekennzeichnet, daß es das Beschichten der Prothesen oder Organe in Vitro mit einer Verbindung umfaßt, die aus einem Peptid nach einem der Ansprüche 1 bis 9 oder seiner Amid-Form ausgewählt ist.

**33.** Verfahren zum Isolieren des Laminin-Zellenoberflächenrezeptors aus Waschmittelextrakten von Zellen oder von Zellmembranen, die an eine Laminin-Affinitätssäule gebunden sind, dadurch gekennzeichnet, daß es das Zugeben einer aus einem Peptid nach einem der Ansprüche 1 bis 9 und seiner Amid-Form ausgewählten Verbindung zu den Zellen bzw. Zellmembranen umfaßt.

**34.** Träger für metastatische Target-Tumorzellen, dadurch gekennzeichnet, daß er ein Peptid nach einem der Ansprüche 1 bis 10 oder seine Amid-Form enthält.

**35.** Laminin-B-Ketten-spezifischer Antikörper, dadurch gekennzeichnet, daß er einen Antikörper gegen das BSA-Konjugat eines Peptids nach einem der Ansprüche 1 bis 10 aufweist.

**36.** Zusammensetzung, dadurch gekennzeichnet, daß sie die Anhaftung von Zellen an ein Substrat fördert, wenn diese auf dem Substrat immobilisiert sind, und ein Peptid nach einem der Ansprüche 1 bis 9 enthält.

**Revendications**

**1.** Peptides caractérisés en ce qu'ils possèdent une activité similaire à celle de la laminine et qu'ils sont choisis parmi les peptides suivants :
    (1) tyrosine-isoleucine-glycine-sérine-arginine;

(2) cystéine-aspartate-proline-glycine-tyrosine-isoleucine-glycine-sérine-arginine;

(3) aspartate-proline-glycine-tyrosine-isoleucine-glycine-sérine-arginine;

(4) glycine-tyrosine-isoleucine-glycine-sérine-arginine;

et leurs dérivés, ces derniers étant choisis parmi leurs dérivés amide, leurs dérivés conjugués avec des protéines, leurs dérivés cyclisés et leurs dérivés polymérisés.

**2.** Peptide selon la revendication 1, caractérisé en ce qu'il est le peptide tyrosine-isoleucine-glycine-sérine-arginine.

**3.** Peptide selon la revendication 1, caractérisé en ce qu'il est le peptide cystéine-aspartate-proline-glycine-tyrosine-isoleucine-glycine-sérine-arginine.

**4.** Peptide selon la revendication 1, caractérisé en ce qu'il est le peptide aspartate-proline-glycine-tyrosine-isoleucine-glycine-sérine-arginine.

**5.** Peptide selon la revendication 1, caractérisé en ce qu'il est le peptide glycine-tyrosine-isoleucine-glycine-sérine-arginine.

**6.** Peptide selon l'une quelconque des revendication 1 à 5, caractérisé en ce qu'il est fixé à la surface d'une fibre de résine synthétique.

**7.** Peptide selon l'une quelconque des revendications 1 à 5, caractérisé en ce qu'il est fixé à la surface d'un dispositif percutané.

**8.** Peptide selon l'une quelconque des revendications 1 à 5, caractérisé en ce qu'il est couplé à du collagène.

**9.** Peptide selon l'une quelconque des revendications 1 à 5, caractérisé en ce qu'il se présente sous la forme d'une lotion, de pommade, d'un gel, d'un colloïde ou d'une poudre.

**10.** Peptide caractérisé en ce qu'il possède une activité similaire à celle de la laminine et qu'il est le dérivé amide du peptide proline-aspartate-sérine-glycine-arginine.

**11.** Agent à activité anti-métastatique vis-à-vis des cellules tumorales métastatiques, caractérisé en ce qu'il comprend un peptide selon l'une quelconque des revendications 1 à 10 ou son dérivé amide et un support approprié pour un agent anti-métastatique.

**12.** Agent pour inhiber la formation de métastases dans les poumons, caractérisé en ce qu'il comprend un peptide selon les revendications 1 à 10 ou son dérivé amide, en quantité suffisante pour inhiber la formation de métastases dans les poumons.

**13.** Agent pour stimuler la migration des cellules épithéliales d'une lésion de la cornée par exemple, ou d'une autre paroi, caractérisé en ce qu'il comprend un peptide selon l'une quelconque des revendications 1 à 9 ou son dérivé amide, en quantité suffisante pour stimuler une telle migration.

**14.** Agent pour inhiber la formation de structures de type capillaire par les cellules endothéliales, caractérisé en ce qu'il comprend un peptide selon l'une quelconque des revendications 1 à 9 ou son dérivé amide, en quantité suffisante pour empêcher la formation de structures de type capillaire.

**15.** Agent pour bloquer la formation de nouveaux vaisseaux sanguins, caractérisé en qu'il comprend un peptide selon l'une quelconque des revendications 1 à 9 en quantité suffisante pour bloquer la formation de nouveaux vaisseaux sanguins, et un support approprié.

**16.** Agent pour bloquer le développement des vaisseaux sanguins dans les tissus, caractérisé en ce qu'il comprend un peptide selon l'une quelconque des revendications 1 à 9, en quantité suffisante pour bloquer le développement des vaisseaux sanguins, et un support approprié.

17. Agent pour traiter le sarcome de Kaposi, caractérisé en ce qu'il comprend un peptide selon l'une quelconque des revendications 1 à 10 en quantité suffisante pour traiter le sarcome de Kaposi.

18. Agent pour favoriser une meilleure adhérence des cellules épithéliales ou endothéliales sur des prothèses vasculaires ou autres organes artificiels, caractérisé en ce qu'il comprend un peptide selon l'une quelconque des revendications 1 à 9 ou son dérivé amide.

19. Facteur d'anti-adhérence des cellules épithéliales sensibles à la laminine, caractérisé en ce qu'il comprend un composé choisi parmi les peptides selon l'une quelconque des revendications 1 à 9, leurs dérivés amide et leurs dérivés conjugués à la SAB.

20. Facteur d'anti-adhérence des cellules épithéliales sensibles à la laminine, caractérisé en ce qu'il comprend un anticorps dirigé contre le dérivé conjugué d'un peptide selon l'une quelconque des revendications 1 à 9, et de la SAB ou d'un autre composé.

21. Facteur de stimulation de la migration des cellules épithéliales, caractérisé en ce qu'il comprend un composé choisi parmi les peptides selon l'une quelconque des revendications 1 à 9, leurs dérivés amide et leurs dérivés conjugués à la SAB.

22. Facteur d'inhibition de la migration des cellules épithéliales sensibles à la laminine, caractérisé en ce qu'il comprend un composé choisi parmi les peptides selon l'une quelconque des revendications 1 à 9, leurs dérivés amide et leurs dérivés conjugués à la SAB.

23. Support actif vis-à-vis de la stimulation de la fixation des cellules épithéliales, endothéliales ou neurales, caractérisé en ce qu'il comprend un composé choisi parmi les peptides selon l'une quelconque des revendications 1 à 9, leurs dérivés amide et leurs dérivés conjugués à la SAB.

24. Support présentant une surface traitée, caractérisé en ce qu'il comprend un peptide selon l'une quelconque des revendications 1 à 9, ce dernier étant fixé à la surface d'un support solide de manière à ce que les cellules se fixent sur ledit support.

25. Support présentant une surface traitée selon la revendication 24, caractérisé en ce que le support est choisi parmi le verre, une résine synthétique, et un polysaccharide à longue chaîne.

26. Substrat présentant une surface traitée selon la revendication 24, caractérisé en ce que le support est choisi parmi la nitrocellulose et les polyesters.

27. Support présentant une surface traitée selon la revendication 24, caractérisé en ce que le support est de l'agarose.

28. Prothèse caractérisée en ce qu'elle présente une surface biologiquement active qui possède une activité vis-à-vis de la fixation cellulaire, ladite surface étant liée à un peptide selon l'une quelconque des revendications 1 à 9.

29. Prothèse selon la revendication 28, caractérisée en ce que ladite surface constitue une partie d'un greffon vasculaire.

30. Prothèse selon la revendication 28, caractérisée en ce que ladite surface est faite d'une fibre de résine synthétique.

31. Prothèse selon la revendication 28, caractérisée en ce que ladite surface comprend une partie d'un dispositif percutané.

32. Procédé pour favoriser une meilleure adhérence des cellules épithéliales ou endothéliales, vis-à-vis de prothèses vasculaires et autres organes artificiels, caractérisé en ce qu'il comprend l'étape de recouvrement in vitro des prothèses ou organes avec un composé choisi parmi les peptides selon l'une quelconque des revendications 1 à 9 et leurs dérivés amide.

**33.** Procédé pour isoler le récepteur de surface cellulaire vis-à-vis de la laminine, à partir d'extraits de lavage de cellules ou de membranes cellulaires liées à une colonne présentant une affinité vis-à-vis de la laminine, caractérisé en ce qu'il comprend l'étape d'addition auxdites cellules ou auxdites membranes cellulaires d'un composé choisi parmi les peptides selon l'une quelconque des revendications 1 à 9 et leurs dérivés amide.

**34.** Vecteur pour cibler les cellules tumorales métastatiques, caractérisé en ce qu'il comprend un peptide selon l'une quelconque des revendications 1 à 10 ou son dérivé amide.

**35.** Anticorps spécifique de la chaîne B de la laminine, caractérisé en ce qu'il comprend un anticorps dirigé contre le dérivé conjugué à la SAB d'un peptide selon l'une quelconque des revendications 1 à 10.

**36.** Composition caractérisée en ce qu'elle stimule la fixation des cellules sur un support quand elle est immobilisée sur ledit support et qu'elle comprend un peptide selon l'une quelconque des revendications 1 à 9.

# FIG.I

## Reaction of Peptide Specific Antibodies with Laminin

**A.**

Relative Titer

Peptide
Laminin

P1  P2  P3  P4  P5  P6  P7

Antisera

**B.**

Laminin/Peptide (%)

1  2  3  4  5  6  7

Antipeptide Antibody

EP 0 278 781 B1

LAM Pi P1 P2 P3 P4 P5 P6 P7

200K

116K

92K

68K

FIG.IB

**Effect of Sequence Specific Antibodies on HT 1080 Cell Attachment to Laminin Substrate**

FIG.2

FIG.3

FIG. 4

Attachment of HT 1080 and CHO Cells to Peptide Conjugates

A. HT 1080 Cells

B. CHO Cells

Inhibition of HT 1080 Cell
Attachment to Laminin Substrate
By Peptide Conjugates

FIG.5

FIG.6A

FIG.6B

FIG.7

FIG.8

# CELL MIGRATION ASSAY

FIG.9

PULMONARY METASTASES (%)

FIG.10

FIG.11

FIG.12A

FIG.12B

FIG.12C

FIG.13

INHIBITION OF INVASION BY LAMININ PEPTIDES